# EUROPEAN PATENT SPECIFICATION

(11) **EP 4 712 854 B1**
(45) Date of publication and mention of the grant of the patent: **30.09.2026**
(21) Application number: 24739176.6
(22) Date of filing: 02.07.2024
(51) Int. Cl.: A61B 5/279, A61B 5/391, A61B 5/00, A61B 5/1455, A61B 5/1459, A61B 17/425

(54) **PROBE WITH SENSOR**
SONDE MIT SENSOR
SONDE AVEC CAPTEUR

(30) Priority: 14.07.2023 EP 23382725
(43) Date of publication of application: 25.03.2026
(73) Proprietor: Sonda Devices S.L., 46004 Valencia (ES)
(72) Inventor: ALBEROLA RUBIO, José, 46017 VALENCIA (ES); PELLICER MARTINEZ, Antonio, 46004 VALENCIA (ES)
(74) Representative: ZBM Patents - Zea, Barlocci & Markvardsen
(86) International application number: PCT/EP2024/068573
(87) International publication number: WO 2025/016721

(56) References cited:
- EP-A2- 1 764 034
- WO-A1-2019/196504
- WO-A1-2022/189843
- US-A1- 2023 013 816
- BAYLIS MEDICAL: "Complex Diagnostic Mapping Solutions ENHANCED DIAGNOSTIC PRECISION FOR CORONARY SINUS MAPPING AND BEYOND", 1 January 2020 (2020-01-01), XP093103164, Retrieved from the Internet <URL:https://www.baylismedical.com/system/resource_files/PRM-00493_EN_EPstar_Diagnostic_Mapping_Solutions_Brochure_J-1_V-4.pdf> [retrieved on 20231120]
- GEORGIOU JULIUS ET AL: "The ElectroUteroGraph: A Novel Tool for Assessing Uterine Contractions of Non-Pregnant Women", vol. 3, 15 March 2022 (2022-03-15), pages 34 - 40, XP093103021, Retrieved from the Internet <URL:https://ieeexplore.ieee.org/ielx7/8782705/9698118/09735315.pdf?tp=&arnumber=9735315&isnumber=9698118&ref=aHR0cHM6Ly9pZWVleHBsb3JlLmllZWUub3JnL2Fic3RyYWN0L2RvY3VtZW50Lzk3MzUzMTU=> DOI: 10.1109/OJEMB.2022.3159097

## Description

### FIELD OF THE DISCLOSURE

The present disclosure relates to devices to measure uterine activity.

### BACKGROUND ART

There are currently no known effective techniques or means for monitoring comprehensive uterine activity of a non-pregnant uterus. Document EP1764034 discloses implantable self-calibrating optical sensors. Document WO2019196504 describes a multi-sensor composite right ventricle electrode and an integrated self-adaptive adjustment method for cardiac pacing frequency. Document US2023013816A1 describes probes, systems and methods for measuring and/or characterizing uterine activity in a non-pregnant uterus. Document WO2022/189843 describes a device for the measurement of endouterine parameters.

lt is an object of the present disclosure to provide probe(s), system(s), and methods for acquiring activity information of the non-pregnant uterus, and for characterizing said activity.

### SUMMARY

In an aspect of the disclosure there is provided a probe or catheter according to claim 1. The catheter is for measuring both electrical and blood activity or physiological activity of a uterus or a uterine muscle in a non-pregnant uterus. The probe comprises a first assembly of electrodes, and one or more PPG -photoplethysmography- sensors; the first assembly of electrodes comprises a first electrode, the first electrode being a ring sensing electrode, concentrically arranged around the catheter; and a second electrode, the second electrode being a ring sensing electrode, concentrically arranged around the catheter; wherein the first electrode and the second electrode are separated along a longitudinal axis (A) of the catheter by a first interelectrode separation (IE); and wherein the second electrode is arranged at a distal separation (DS) from a distal end of the catheter. The one or more PPG sensors are arranged at least on one side of a surface of the catheter and/or at least embedded in the catheter. The one or more PPG sensors may be, one or more of them, arranged at a distance from the distal end from 0 mm to 15mm. One or more PPG sensors may be installed or arranged between two electrodes. Photoplethysmography, known commonly as PPG, may be defined as an optical technique to detect blood volume changes in a microvascular bed of tissue. The measures obtained by the one or more PPG sensors of the disclosure may correlate to the degree of oxygenation a tissue in the uterine cavity. The electrodes may provide uterine peristalsis and myometrial activity information where uterine peristalsis and myometrial activity are terms used to refer to contractions of the uterine muscle, which are associated with electrical activity of the muscle. The uterine myometrium may be subject to variation in its contractile activity, which may vary from individual to individual and which may depend on factors such as the menstrual cycle, pathologies, and/or pregnancy.

The PPG sensor may comprise a light source and a photodetector. The light source may comprise for example an infrared light emitting diode -IR-LED- or a green LED, or a blue LED, or red LED. IR-LEDs may be used for measuring flow of blood that is more deeply concentrated in certain parts of body such as the muscles, whereas green LED may be used for calculating the absorption of oxygen in oxyhemoglobin (oxygenated blood) and deoxyhemoglobin (blood without oxygen present). Green LED light penetrates more deeply into tissue and therefore can provide measurements that are more accurate. Other LEDs with different colors may be used to illuminate hemoglobin. PPG sensors also use a photodetector to measure the intensity of reflected light from the tissue. The blood volume changes can then be measured (calculated) based on the amount of the detected light. PPG sensors are also useful in the determination of hyperemia, or an excess of blood flow. The PPG sensors allow for monitoring light absorption by a tissue's blood and may provide information about parameters in blood, such as glucose or other parameters related to blood.

The catheter is configured such that, in use, at least one of the one or more PPG sensors faces and is substantially in contact with at least part of a uterine internal surface or a myometrial wall and/or a myometrial cavity of the subject, and such that at least the first electrode and the second electrode contact at least part of a uterine internal or a myometrial wall and/or a myometrial cavity of the subject. In examples, the at least a part of the surface of the catheter or part of the catheter itself may be transparent or substantially transparent with a certain degree of opacity, for example between 1% and 50 % opacity.

Advantageously a catheter as provided in the present disclosure allows for identification or characterization of timely and locally correlated uterine electrical activity and blood activity. Physiological activity or blood activity or physiological activity is to be understood as tissue oxygenation, blood volume in the uterine internal surface, hemodynamics, or other similar activity.

The catheter's diameter may range from 2,33 mm (7 FR) to 5 mm (15 FR) or from 2,33 mm (7 FR) to 7 mm (21 FR).

On the one hand, some medications cause greater oxygenation of a uterine surface tissue, which may be positive for some treatments, the treatments including administering painkillers for dysmenorrhea, embryo implantation, or others. On the other hand, a certain peristaltic - mechanical-activity of the uterus may be considered optimal for some treatments. A combination of information about mechanical activity of the uterus and physiological information of the uterus, such as oxygenation of tissues or parameters -for example glucose- levels provides valuable information which may be used for evaluation of correlations between both activities or derived parameters such that treatments may be adjusted individually depending on the subject and depending on conditions influencing the response to each treatment.

A method of use of the catheter of the present disclosure is not covered by the claims. The method may comprise a diagnostic method of the physiological activity of the uterus, a method for determining an optimal time for embryo transfer, a method for development of treatments to improve one or more of: embryo transfer rate, reduction of dysmenorrhea, adenomyosis diagnostics, fibromas, endometriosis, RIF -recurrent implantation failure- and/or methods to evaluate surgical or presurgical or postsurgical procedures.

The catheter is configured to particularly assist in assisted reproductive techniques. The catheter is referred to as "assisted reproductive technique catheter" and is configured to measure uterine activity during a pre-transfer time period or during transfer of semen or of an embryo containing solution into a uterus. The catheter comprises a lumen configured to receive an internal catheter for the passage of an embryo-containing cannula or direct passage of an embryo or embryos and configured to receive and allow the passage a liquid substance. Assisted reproductive technique comprises fertility treatments in which sperm or embryos are handled, for example, artificial Insemination (AI) or Intrauterine Insemination (IUI) which comprises depositing a semen sample, which has been prepared in advance in the laboratory, inside a woman's uterus in order to increase the potential of the spermatozoa and improve the chances of the egg being fertilized. Assisted reproductive technique comprises embryo transfer after an in-vitro fertilization, which comprises joining an egg with sperm in laboratory -in vitro- and then transferring an embryo resulting from the joining to a mother's uterus, possibly leading to a pregnancy. The assisted reproductive technique comprises a step in which a transfer of either semen or an embryo is performed. The catheter may be made of a biocompatible material, for example a biocompatible plastic.

The assisted reproductive technique catheter of the disclosure is configured to perform the transfer. For such transfer, the lumen is provided which is configured to receive an internal catheter for the passage of an embryo-containing cannula or direct passage of an embryo or embryos and configured to receive and allow the passage a liquid substance, for example semen. The lumen comprises an inner diameter of the lumen equal or greater than 7 FR in the French scale, or equal or greater than 2,33 mm to allow passage of an embryo. An assisted reproduction technology, for example artificial insemination or in-vitro fertilization (IVF) and its derivatives, may comprise a placement of sperm or semen or embryos in a uterus of a female, possibly leading to an ongoing pregnancy. During placement of the embryos, the catheter of the present example may be inserted through the cervix into a uterine cavity. A further internal catheter may be inserted through the outer catheter, and its distal end may protrude from the outer catheter. The inner catheter is then positioned, to a point near the fundus, or top, of the uterine cavity. Finally, embryos and culture fluid for nourishment may be flushed through the inner catheter. All catheters may be finally withdrawn.

The catheter with lumen provides a means for monitoring the uterine peristaltic activity with high degree of reliability before or during an embryo placement or embryo transfer occurs.

Advantageously an assisted reproductive technique catheter as provided in the present disclosure allows for identification or characterization of uterine electrical and physiological activity during a pre-transfer time period or during transfer of semen or an embryo containing solution into a uterus.

The catheter of the disclosure comprises PPG sensors and electrodes which provides for local and temporal correlation of parameters. The catheter of any one of the examples of the disclosure may represent a tool for obtaining correlated uterine parameters, such as peristaltic activity, hemodynamics, glucose, or others. Such correlated uterine parameters may be used for training neural networks or for performing statistical analysis in other to obtain one or more parameters which may be considered relevant for the customization of treatments related to the menstrual cycle or for assisted reproductive techniques.

In any of the examples of the present disclosure, the electrodes may be made of an iridium palladium alloy. The outer diameter of the electrodes may be equal or greater than 6 FR in the French scale or equal or greater than 2 mm The outer diameter of the electrodes may be greater than a diameter of the catheter ranging from 2,33 mm (7 FR) to 5 mm (15 FR) or from 2,33 mm to 7 mm (21 FR). For example, the outer diameter of the electrodes may be comprised in the range (7,001 FR - 21,001 FR).

In any of the examples of the present disclosure the distal separation DS may comprise a distance between 0 mm and 10mm, including 0 and 10 mm or the distal separation DS may be at least of 0,5 m, or 1 mm or 2 mm, or 3 mm, or the distal separation DS may be equal or less than 10 mm.

In any of the examples of the present disclosure the catheter may be flexible. The catheter as described herein may be inserted in a vagina and displaced until the distal end of the catheter reaches the cervical canal and uterine cavity. The catheter may have a length of between 20 and 50 cm. The lumen may present an inner diameter of the lumen equal or greater than 7 FR in the French scale, or equal or greater than 2,33 mm. An embryo or a solution containing an embryo is advantageously allowed to pass through and be inserted into a uterus.

The first assembly of electrodes comprises a first electrode, where the first electrode may be a ring sensing electrode concentrically arranged around any of the example catheters of the disclosure; and a second electrode, the second electrode may be a ring sensing electrode concentrically arranged around any of the example catheters of the disclosure. Uterine or myometrial peristalsis can be detected by processing the signal provided by the electrodes. Bipolar electrodes provide the advantage of providing located information about the myometrial walls. In some examples, two or more assemblies of electrodes are provided in any of the example catheter of the disclosure. In particular, when providing assemblies of two bipolar electrodes allows that signal processing the signals received from both electrodes may provide information which is more precise in terms of information per mm2 area of a myometrial wall.

The electrodes in each assembly are separated along a longitudinal axis of the catheter by a first interelectrode separation IE; and one of the electrodes, for example the second electrode, is arranged at a distal separation DS from a distal end of the catheter. In some examples the distal separation is substantially zero, whereas in other examples the distance separation is at least of 3 mm. The distal separation may be configurable by the user. The assembly of electrodes may be configured to be movable or displaceable. The catheter may comprise displacement means so that the catheter is configured to position the electrodes at a distal separation DS, chosen before or during use, from the distal end of the catheter. For example, the displacement means may comprise an inner helical machining of the electrodes, e.g., in the form of nuts, and the outer surface of the catheter, e.g., in the form of a screw, to displace the electrodes screwing them in a direction or an opposite direction depending on the separation to be obtained. For example, the displacement means may comprise an outer surface made of a material on which the electrodes may slide or glide. The displacement means may comprise a grove made in the outer surface of the catheter and a flap or flange in the electrodes, such that the flap may be displaced along the grove. The electrodes are configured to sense the myometrial activity when positioned inside a uterus. A practitioner may choose a distance IE or DS in which the electrodes need to be positioned depending on the anatomy of a woman's uterus or depending on the technique, for example assisted reproductive technique, to be performed. The displacement means may be configured to let the electrodes be displaced by application of a displacement force and pressure, for example applied by a user's fingers, and at the same time the displacement means may be configured to not let the electrodes be displaced or configured to maintain the electrodes in a desired position when the catheter is passed through the cervix. In other words, the displacement means may be configured to let the electrodes be displaced when a predetermined displacement pressure and/or force are applied to the electrodes. The predetermined displacement pressure and/or force are greater than the pressure and/or force the cervix applies to a catheter passing through. The IE and the DS as seen, may be configurable or may be fixed in manufacture. When configurable, and during use, an IE and a DS may be adjusted depending on the use or final technique, for example assisted reproductive technique, to be performed and, in examples, considering an interelectrode separation, IE, of 1 mm or above.

Advantageously, any of the example catheters of the disclosure are configured to be used for detecting peristalsis and physiological activity or hemodynamics or blood activity by measuring electrical properties of a uterus or myometrium, for example right before or during or even after a transfer of a solution containing reproductive agents including any of semen, or sperm containing solution or embryo containing solution or solution to push an embryo attached to the distal end of the catheter, into a woman's uterus. The assisted reproductive technique catheter provides a reliable device to acquire signals which may be analyzed before or during the transfer of a solution containing reproductive agents.

**In** a further aspect of the disclosure, not covered by the claims, there is provided a method for identifying a time when a likelihood of success of a medical treatment of a subject over a predetermined threshold is obtained, the method comprising positioning a catheter according to the present disclosure within a uterine cavity of the subject; wherein positioning comprises positioning the catheter such that at least one of the one or more PPG sensors faces and is substantially in contact with at least part of a uterine or myometrial wall and/or a myometrial or uterine cavity of the subject, and such that the first electrode and the second electrode contact at least part of a uterine or myometrial wall and/or a uterine or myometrial cavity of the subject; receiving, from at least one PPG sensor one or more signals representative of a physiological activity or blood activity of the uterine cavity, for example blood activity or hemodynamics of the myometrium; receiving from the first ring sensing electrode and from second ring sensing electrode, electrical signals representative of an electro-physiological state of the uterine cavity; and identifying a time when a likelihood of success of a medical treatment of a subject over a predetermined threshold is obtained based at least in part on the signals obtained from at least one PPG sensor and the signals obtained from the electrodes.

In the present disclosure, the feature "at least one of the one or more PPG sensors faces and is substantially in contact with at least part of a uterine or myometrial wall and/or a myometrial or uterine cavity of the subject" comprises that the PPG sensor is in position such that, in use, at least one of the one or more PPG emits light into a uterine wall and receives reflected light such that a measure related to the reflection of the light into the blood or measure related to the absorbance can be determined. In examples, the PPG sensor may be in contact with the uterine wall; in other examples the PPG sensor may be embedded in the catheter and the catheter is in contact with the uterine wall. In examples where the PPG sensor may be embedded in the catheter and the catheter is in contact with the uterine wall, at least a part of a surface of the catheter or at least a part of the catheter may be transparent or substantially transparent with a certain degree of opacity, for example between 1% and 50 % opacity, such that the light emitted and the light received by the PPG sensor may pass through the surface. In such examples the PPG sensor, in use, may face the uterine wall.

In examples, the method for identifying a time when a likelihood of success of a medical treatment of a subject over a predetermined threshold is obtained comprises using a catheter comprising a lumen, the lumen being configured to receive an internal catheter for a passage of an embryo-containing cannula or direct passage of an embryo or embryos and comprises that the medical treatment is an assisted reproductive technique. In the example, the method comprises identifying a time when a likelihood over a predetermined threshold of assisted reproductive technique success is obtained based at least in part in the signals obtained from the electrodes and on the signals obtained from the PPG sensors. One or more measures related to the reflection of the light into the blood or measure related to the absorbance may comprise bilirubin levels and/or hemoglobin related measures such as oxyhemoglobin, and/or deoxyhemoglobin, and/or methemoglobin, and/or carboxyhemoglobin, and/or sulfhemoglobin and/or glycated hemoglobin.

A predetermined threshold may be established by a practitioner and/or may be based on combination of parameters obtained or calculated based on the received signals from the electrodes and/or the received signals from the PPG sensor(s).

Positioning the catheter within a uterine cavity of the subject, may comprise setting both electrodes and at least one of the PPG sensor(s) at least facing and substantially in contact with at least a part of a myometrial wall and/or a myometrial cavity of the subject at a depth corresponding to any point along the myometrial wall necessary to analyze electrical signals from the electrodes and measurements or signals from the PPG sensor(s). As the first electrode and second electrode of the catheter of the disclosure are known to be at an interelectrode separation, IE, plus a distal separation, DS, from the distal end of the catheter and at a distal separation DS from the distal end of the catheter respectively, a position in the uterus may be chosen so as to select the points from which one or more electrical signals is to be received.

Receiving, from at least the first and second electrodes, electrical signals representative of the electro-physiological state of the myometrium or uterine cavity may comprise receiving a first electrical signal from a first electrode and receiving a second electrical signal from the second electrode. Receiving electrical signals may comprise receiving a first digital data and a second digital data. Receiving electrical signals may comprise receiving electrical signals during 2 minutes or during 3 minutes or during a larger time window of, for example, 10 minutes. The capture of electrical signals produced by muscles during a muscle contraction is known as electromyography. The electrical signals are generated by the exchange of ions across the membranes of muscle fibers due to muscle contraction. Electromyography -EMG- is the acquisition, recording and analysis of electrical activity generated in nerves and muscles through the use of electrodes, for example surface electrodes. Electro hysterogram EHG or EHG-IC -internal cavity- are examples of EMG in the present disclosure. The measurements extracted from EHG provide valuable information about physiology and muscle activation patterns. In the case of the disclosure, EHG is advantageously used by a catheter which may be used for the transfer of a solution containing reproductive agents.

Receiving, from at least one PPG sensor one or more signals representative of a physiological activity or blood activity of the uterine cavity, for example blood activity of the myometrium, may comprise receiving electrical signals representative of the changes in light absorption due to oscillations in arterial blood volume.

Identifying a time window with a likelihood, for example the highest likelihood, of assisted reproductive technique success may be based on the electrical signals provided by the electrodes and on the blood or physiological activity -electrical- signals provided by the one or more PPG sensor(s). Identifying a time window with a likelihood of assisted reproductive technique success may comprise identifying features appropriate for an assisted reproductive technique, such as an appropriate contraction frequency of the myometrial wall, a signal frequency average sensed by the electrodes, oxygen saturation of the myometrium, glucose, or others. Some example features may comprise mean frequency, frequency median, standard deviation, ratio of high and low frequencies, first unnormalized moment, RMS, and entropy, or one or more of a contraction frequency, peak contraction intensity, mean contraction intensity, median contraction intensity, median contraction duration, mean contraction duration, peak contraction duration, shortest contraction duration, lowest contraction intensity, direction of propagation of a peristaltic wave, mean direction of propagation of a peristaltic wave, median direction of propagation of a peristaltic wave, peristaltic wave origin, mean peristaltic wave origin, median peristaltic wave origin, one or more features related to the differences between the peristaltic activity in different points of time, entropy, intensity/amplitude RMS, first normalized moment, contraction frequency standard deviation, ratio between high and low contraction frequencies, oxygenation, SPO₂, bilirubin level and/or hemoglobin related measures such as oxyhemoglobin, and/or deoxyhemoglobin, and/or methemoglobin, and/or carboxyhemoglobin, and/or sulfhemoglobin and/or glycated hemoglobin. or any combination thereof, and/or any statistical representation of any order of the aforementioned features or any combination thereof. Contraction herein refers to uterine muscle contraction or peristaltic activity of the uterus or the myometrium. Any of the aforementioned features, and any combination thereof may be used to detect a contraction or a contraction event in the received electrical signals from the first and the second electrodes. For example, threshold values for each of the aforementioned features may be established, such that, a contraction or contraction event is positively detected based on a comparison between the measured or calculated value of the feature and the threshold value established for the feature. Thus, a discrete numerical and/or categorical characterization of the peristaltic activity may be obtained.

The advantages of a method according to the disclosure are varied and comprise the measurement, through the application of EHG and PPG techniques, of representative signals of the myometrium to assist and to identify a point of time when a medical treatment or a transfer of a solution containing reproductive agents should be performed to obtain a probability or likelihood, for example the highest probability, of medical treatment success or sperm or embryo transfer success. A probability of transfer success may comprise a probability of pregnancy. The fact that the catheter of the disclosure need not be removed after the EHG-PPG and before the transfer, provides the further advantage that the cervix is not manipulated, or that the cervix remains right in the same conditions as when the electrical signals provided by the electrodes (regardless of the signal delay and signal processing delay, which may be considered insignificant or of little relevance for the technique) are analyzed and thus, the cervix remains in the same conditions as when identifying of a time window with the a likelihood of assisted reproductive technique success.

Compared to prior art solutions where ultrasounds are performed to check the status of a uterus before a transfer and after some minutes or hours the transfer is performed, the catheter and method according to the present disclosure improves the current techniques.

### BRIEF DESCRIPTION OF THE DRAWINGS

Non-limiting examples of the present disclosure will be described in the following, with reference to the appended drawings, in which:
Figure 1 schematically illustrates a catheter not covered by the claims.
Figure 2 schematically illustrates a catheter not covered by the claims.
Figure 3 schematically illustrates a catheter not covered by the claims.
Figure 4 schematically illustrates a catheter not covered by the claims.
Figure 5 illustrates the example of figure 4 where a front view of the cut along line AB is shown.
Figure 6 schematically illustrates a system 6 comprising an assembly of electrodes and a bio amplifier.
Figure 7 schematically illustrates a system 6 comprising a catheter 7 comprising two assemblies of electrodes, a bio amplifier and PPG sensors.
Figure 8 schematically illustrates an assisted reproductive technique catheter.
Figure 9 schematically illustrates an assisted reproductive technique catheter.
Figure 10 schematically illustrates an assisted reproductive technique catheter.
Figure 11 illustrates the catheter of figure 4 further comprising a lumen.
Figure 12 represents an example of a method according to the disclosure.
Figure 13 schematically illustrates an assisted reproductive technique catheter.
Figure 14 schematically illustrates the assisted reproductive technique catheter.
Figure 15 schematically illustrates the assisted reproductive technique catheter.
Figure 16 illustrates the catheter of figure 14 further comprising a coating.
Figure 17 illustrates the example of figure 16 where a front view of the cut along line AB is shown.
Figure 18 schematically illustrates a system comprising an assembly of electrodes and a bio amplifier.
Figure 19 schematically illustrates a system comprising a catheter comprising two assemblies of electrodes.
Figure 20 represents an example of a method 200 not covered by the claims.

### DETAILED DESCRIPTION OF EXAMPLES

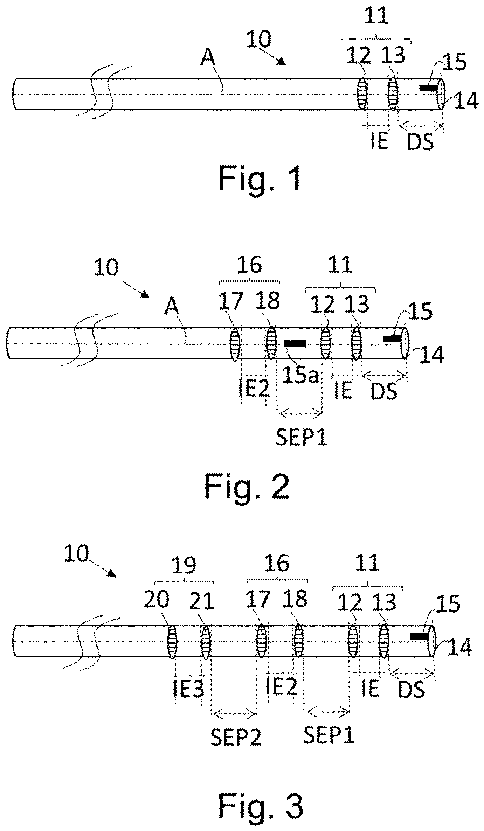

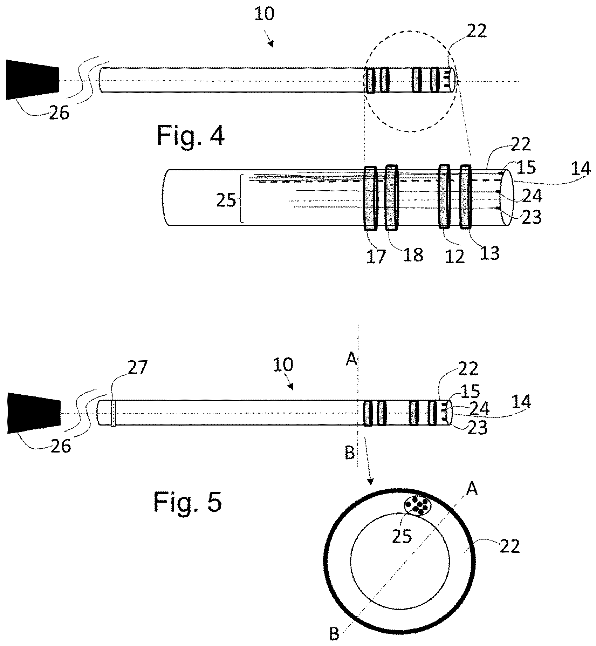

Figure 1 schematically illustrates a catheter 10 comprising a first assembly 11 of electrodes, comprising: a first electrode 12, the first electrode 12 being a ring sensing electrode concentrically arranged around the catheter 10; and a second electrode 13, the second electrode 13 being a ring sensing electrode concentrically arranged around the catheter 10; wherein the first electrode 12 and the second electrode 13 are separated along a longitudinal axis A of the catheter 10 by a first interelectrode separation IE; IE may be comprised in a range from 1 mm to 5 mm. The second electrode 13 is arranged at a distal separation DS from a distal end 14 of the catheter 10. DS may be comprised in a range of 0 mm to 10 mm. The catheter 10 further comprises a PPG sensor 15 between the distal end 14 and the first assembly. The electrodes have a ring portion, which portions may be monolithically formed or separately integrated. The electrical activity sensed by one or more of the electrodes may be output via the catheter's wires as an analog signal. Thus, the catheter generates a set of analog signals during a measurement session. A catheter comprising two electrodes, as shown in Figure 1, may generate a set of two analog signals during a measurement session. The analog signals may be transmitted to a display means, such as a monitor or screen, for visualization, which may be referred to as an electromyogram. The PPG sensor 15 may comprise a light source and a detector. The detector may generate an electrical signal or analog signal which may be transmitted through one or more of the set wires 25, as will be shown with reference to figure 4. Analog signals may be transmitted to a processor. In order to transmit the analog signals generated by the electrodes to a processor and/or to visualization means, a connector, for example, a REDEL connector or another suitable connector, may be attached to the catheter. Further PPG sensors may be distributed around a circumference formed by the catheter's section for example 3 or 4 PPG sensors may be distributed around the circumference. The 3 or 4 PPG sensors may be distributed around the circumference and all of them equidistant from the distal end. In some examples the PPG sensors are substantially equally spaced with each other around the circumference and equidistant from the distal end of the catheter, so that a position where at least one PPG sensor faces at least a part of a uterine cavity wall is facilitated when in use. The "substantially equally spaced with each other" may comprise that each one of the PPG sensors represents a point in the circumference section of the catheter, the points defining different radius forming central angles of 90 degrees or central angles of 120 degrees. Any of the examples of the figures 2 to 20 below may comprise a distribution of two or more PPG sensors around a circumference section of the catheter as explained.

Figure 2 schematically illustrates a catheter 10 comprising the same features as the catheter of figure 1 and further comprising a second assembly 16 of electrodes and an additional PPG sensor 15a, wherein the second assembly 16 of electrodes comprises at least: a third electrode 17, the third electrode being a ring sensing electrode concentrically arranged around the catheter 10; and a fourth electrode 18, the fourth electrode being a ring sensing electrode concentrically arranged around the catheter 10; wherein the third electrode 17 and the fourth electrode 18 are separated by a second interelectrode separation IE2; and wherein the second assembly 16 is arranged at a first separation SEP1 from the first assembly 11, wherein the first separation SEP1 is comprised in a range from 3 mm to 7 mm. IE may be different from IE2, and IE2 may be comprised in a range from 1 mm to 5 mm. The first separation SEP1 comprises a distance from the fourth electrode 18 (comprised in the second assembly 16) to the first electrode 12 (comprised in the first assembly 11). The separation SEP1 may be configured to detect a contractile wave of interest. The separation between assemblies of electrodes allows sensing points in the uterine cavity which are separated by a controlled or configurable distance, SEP1, so that the obtained signal is representative of the muscular activity of exact points in the uterine cavity. The additional PPG sensor 15a is shown between the first assembly 11 and the second assembly 16. The additional PPG sensor 15a may be embedded in the catheter 10 and the catheter may be in contact with the uterine wall, in which case a transparent or translucid surface of the catheter, at least in the part where the additional PPG sensor 15a is embedded, advantageously allows the light to pass through so that the additional PPG sensor 15a can emit and receive light to and from a tissue. The first PPG sensor 15 may also be either embedded in the catheter or arranged on a surface of the catheter 10.

Figure 3 schematically illustrates the catheter 10 comprising the same features as the catheter of figure 2, except for the additional PPG sensor 15a, and further comprising a third assembly 19 of electrodes, wherein the third assembly 19 of electrodes comprises at least a fifth electrode 20, the fifth electrode being a ring sensing electrode concentrically arranged around the catheter 10; and a sixth electrode 21, the sixth electrode being a ring sensing electrode concentrically arranged around the catheter 10; wherein the fifth electrode 20 and the sixth electrode 21 are separated by a third interelectrode separation IE3; and wherein the third assembly 19 is arranged at a second separation SEP2 from the second assembly 16 of the catheter 10, wherein the second separation SEP2 is comprised in a range from 3 mm to 7 mm. The second separation SEP2 comprises a distance from the sixth electrode 21, comprised in the third assembly 19, to the third electrode 17 comprised in the second assembly 16.

SEP1 may be different from SEP2. IE3 may be comprised in a range from 1 mm to 5 mm. IE3 may be different from IE and from IE2.

In examples, the first interelectrode separation (IE), and/or the second interelectrode separation (IE2) and/or the third interelectrode separation (IE3), is comprised in a range from 1 mm to 5 mm. The distances may be different from one another.

In examples, at least one of the electrodes is connected by a respective electrical wire to a connector 26 at a proximal end of the catheter. The electrical wires may be made of copper, of optical fiber, for example.

Figure 4 illustrates the catheter 10 of figure 2, except for the additional PPG sensor 15a, further comprising a coating 22, a second PPG sensor 23 and a third PPG sensor 24, the PPG sensors being placed around the distal end 14 of the catheter. The PPG sensors may be spaced irregularly or evenly around the distal end 14; the electrodes 12, 13, 17, 18 is concentrically arranged around the catheter 10 and over the coating 22; and respective electrical wire in the set of wires 25 is arranged along the catheter 10 from a respective sensor, i.e., electrode or PPG sensor, to the connector 26 and is embedded in the coating 22 or arranged between the coating and the catheter. The connector 26 is provided so that the wires can leave the catheter and may connect to an external processing unit. Figure 4 shows a zoomed portion of the catheter 10 showing the set of wires 25 running embedded inside the coating 22.

Figure 5 illustrates the example of figure 4 where a front view of the cut along line AB is shown. The set of wires 25 are shown inside a tubular arrangement in the coating 22.

In examples, one or more of the wires may be comprised in between the coating and the catheter or embedded in the coating in a loose manner, or loosely embedded or embedded with certain tolerance to be displaced so that, when the electrodes are displaced for varying any of DS, SEP, SEP2, IE, IE2, the wires may provide a certain tolerance for movement. The length of a wire between an electrode and the connector 26 may vary and may allow the electrode to be moved in a range of 1 mm to 3 mm both included.

ln examples where DS, SEP, SEP2, IE, IE2, are fixed or predetermined, and the catheter is not configured to move, the wires may be tightly embedded or arranged between the coating and the catheter or the wires may not be allowed to be moved. In those examples, the wires may be tightly embedded or arranged between the coating and the catheter or the wires may also be loosely embedded or embedded with certain tolerance to be displaced.

ln some examples the coating is made of a biocompatible material or plastic. The coating may be flexible and the catheter may be flexible.

In examples, as shown in figure 5, the catheter comprises a stopper 27 configured to be placed, in use and when inserted in a uterus, at the entry of the cervix, configured thereby to stop the catheter 10 and prevent a user to insert the catheter forward during a vaginal insertion of the catheter. The stopper may be positioned at 4 cm or 5 cm from the distal end 14 of the catheter 10. The stopper may be fixed or may be configured to be displaced from substantially 0,5cm to 1cm or 2cm or from 0,5 cm to 5 cm. The stopper 27 may be configured be displaced by application of a displacement force and pressure, for example applied by a user's fingers. The stopper may be displaceable by comprising, for example, an inner helical machining, e.g., in the form of a nut, and the outer surface of the catheter may present, e.g., the form of a screw along the length occupied by the stopper, to displace the stopper screwing the stopper in a direction or an opposite direction depending on the displacement to be obtained. The stopper 27 may be comprised in any of the examples of the disclosure.

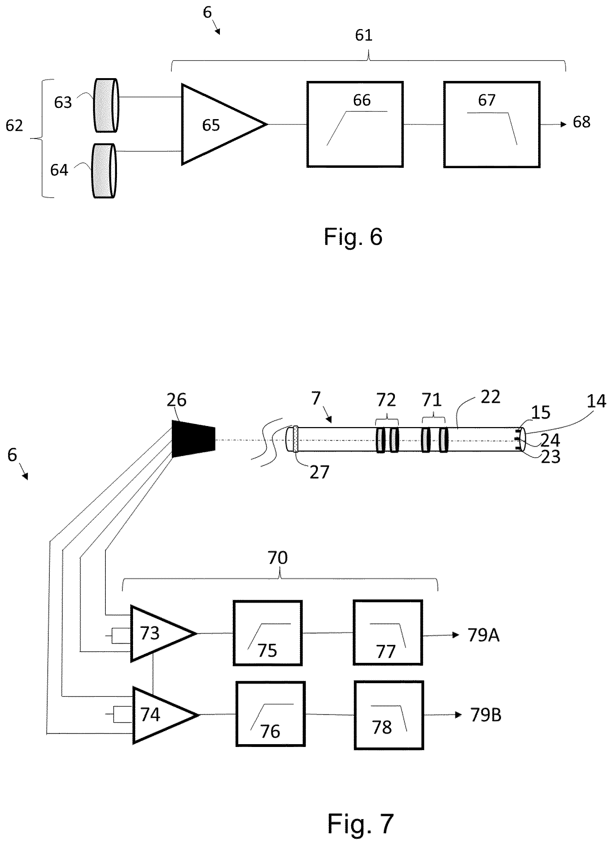

Figure 6 schematically illustrates a system 6 comprising an assembly of electrodes and a bio amplifier 61. For illustrative purposes, the electrodes are shown not arranged around the catheter of the disclosure. In some examples, as illustrated in figure 6, the bio amplifier 61 comprises at least one operational amplifier 65. The bio amplifier processes the two received signals from the electrodes 63 and 64. Figure 6 represents a schematic example of a first electrode 63 of the catheter in electrical connection with a first input pin of the operational amplifier 65 and a second electrode 64 in electrical connection with a second input pin of the operational amplifier 65. The first electrode and the second electrode belong to a same assembly of electrodes. In examples, as the one illustrated in figure 6, the system further comprises at least one or more of a high-pass filter 66 and/or a low-pass filter 67.

Bipolar electrodes provide the advantage of providing located information about the myometrial walls. Monopolar electrodes may provide more generic information of a muscle. The fact of providing assemblies of two bipolar electrodes is that signal processing the signals received from both electrodes may provide information which is more precise in terms of information per mm2 area of a myometrial wall. A bipolar signal is provided by calculating the difference between the voltage amplitude provided by one of the electrodes minus the voltage amplitude provided by the other electrode. The high-pass filter may comprise a cut-off frequency of 0.1 Hz. The low-pass filter may comprise a cut-off frequency of 25Hz.

Figure 7 schematically illustrates a system 6 comprising a catheter 7 comprising two assemblies 71 and 72 of electrodes, 3 PPG sensors 15, 23 and 24 and a bio amplifier 70. The electrodes are shown arranged around the catheter 7. The wires coming from the assembly 71 may, for example, feed the operational amplifier 73. The result of the difference provided by the operational amplifier 73 between the voltage amplitude signals of the two electrodes of the assembly 71 may be provided to filter 75, which output may feed a further filter 77. The wires coming from the assembly 72 may, for example, feed the operational amplifier 74. The result of the difference between the voltage amplitude signals of the two electrodes of the assembly 72 may be provided to filter 76, which output may feed a further filter 78. The outputs 79A and 79B may be processed to interpret a contractile wave of the myometrial walls for example. Such a contractile wave is represented by the electrical signals provided by the first and second assemblies of electrodes at different heights or points of the uterine cavity. The contractile wave can therefore be represented in two different points of the uterine cavity at the same time. A similar arrangement may be provided for 3 assemblies, and a contractile wave can therefore be represented in three different points of the uterine cavity at the same time. As a contractile wave, theoretically, propagates in a uterine cavity from the fallopian tubes to the cervix, the contractile wave propagation may be followed by the arrangement of assemblies of electrodes in the catheter of the disclosure. The wires coming from the 3 PPG sensors 15, 23, 24 may, for example, feed a transimpedance operational amplifier and an arrangement for representing or processing signals received from the PPG sensors may comprise any of example arrangements in the art.

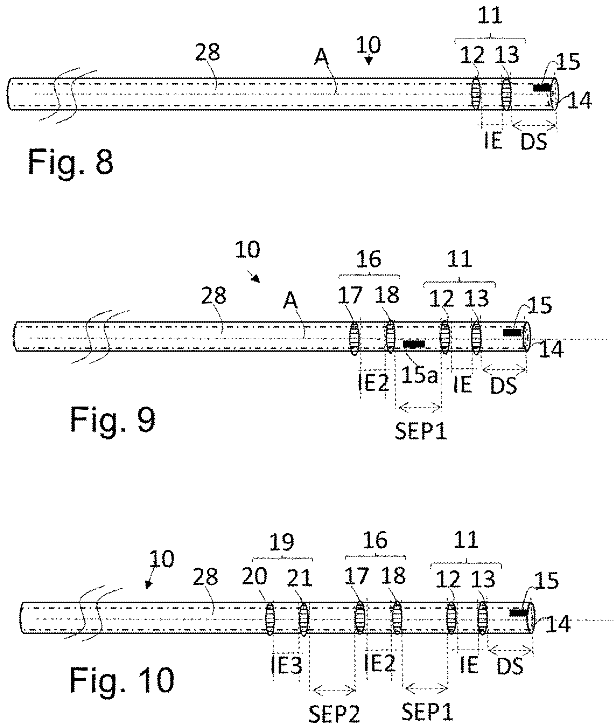

Figure 8 schematically illustrates an assisted reproductive technique catheter 10 which may comprise the catheter 10 of figure 1 further comprising a lumen 28 configured to receive an internal catheter for the passage of an embryo-containing cannula or direct passage of an embryo or embryos and configured to receive and allow the passage a liquid substance. The lumen may be understood as a volume in the inner volume left by the housing of the catheter which is represented in a non-limitative example by dotted lines in the figures.

Figure 9 schematically illustrates an assisted reproductive technique catheter 10 comprising the same features as the catheter 10 of figure 2 and further comprising the lumen 28.

Figure 10 schematically illustrates an assisted reproductive technique catheter 10 comprising the same features as the catheter 10 of figure 3 and further comprising the lumen 28.

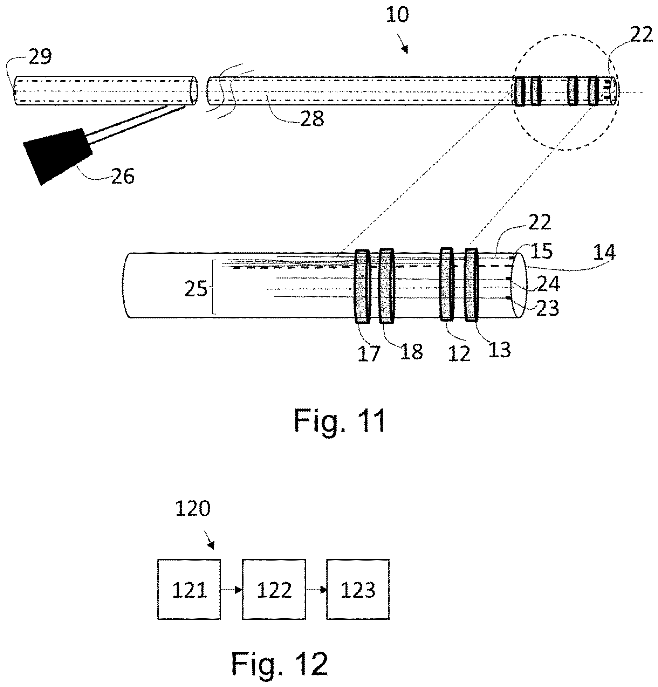

Figure 11 illustrates the catheter of figure 4 further comprising the lumen 28. The catheter 10 further comprises an entry 29 to insert a cannula or syringe or internal catheter to insert a solution containing reproductive agents into the uterus through the catheter. The entry 29 may comprise a flange not shown for attaching an injection syringe or cylinder for the introduction of a solution into the lumen 28. A front view of a cut along a line would be equivalent as the one shown in figure 5.

A bio amplifier as the one shown in figure 7 may be used with the example catheter 10 with lumen 28.

Figure 12 represents an example of a method 120 according to the disclosure for identifying a time when a likelihood of success of a medical treatment over a predetermined threshold of a subject is obtained, the method comprising, in block 121, positioning a catheter 10 within a uterine cavity of the subject, wherein positioning comprises positioning the catheter such that at least one of the one or more PPG sensors faces and is substantially in contact with at least part of a myometrial wall and/or a myometrial cavity of the subject, and such that the first electrode and the second electrode contact at least part of a myometrial wall and/or a myometrial cavity of the subject. In some examples the distance of the first assembly of electrodes from the cervix may be configurable depending on the user or medical treatment needs. The method comprises, in block 122, receiving, from the first and second electrodes, electrical signals representative of the electro-physiological state of the myometrium and receiving, from at least one of the PPG sensor(s), one or more signals representative of the blood activity or hemodynamics or physiological activity of the uterine cavity, for example blood activity state of the myometrium; and the method comprises, in block 123, identifying a time when a likelihood of success of a medical treatment of a subject over a predetermined threshold is obtained based at least in part on the signals obtained from at least one PPG sensor and on the signals obtained from the electrodes.

In some examples of the method, the catheter comprises a lumen, the lumen being configured to receive an internal catheter for a passage of an embryo-containing cannula or direct passage of an embryo or embryos; the medical treatment is an assisted reproductive technique; and the method comprises identifying a time when a likelihood of success of assisted reproductive technique success over a predetermined threshold is obtained based at least in part on the signals obtained from at least one PPG sensor and from the electrodes. The time when a likelihood of success is obtained may comprise a time window with a likelihood over a predetermined threshold of assisted reproductive technique success based at least in part on the signals obtained from at least one PPG sensor and on the signals obtained from the electrodes.

The method for assisted reproductive technique may comprise, after identifying the time when a likelihood over a predetermined threshold of success is obtained, and before removing the catheter from the uterine cavity, performing the assisted reproductive technique using the catheter during at least a time window comprising the identified time with the likelihood. The time window with the highest likelihood may comprise several minutes, or may comprise several seconds, for example, between 1 minute to 5 minutes. The time window may comprise checking during approximately 2 minutes or 3 minutes that predetermined features are optimum and then, transfer a solution containing reproductive material or agents, for example sperm or an embryo. Some example features may comprise mean frequency, frequency median, standard deviation, ratio of high and low frequencies, first unnormalized moment, RMS, and entropy, or one or more of a contraction frequency, peak contraction intensity, mean contraction intensity, median contraction intensity, median contraction duration, mean contraction duration, peak contraction duration, shortest contraction duration, lowest contraction intensity, direction of propagation of a peristaltic wave, mean direction of propagation of a peristaltic wave, median direction of propagation of a peristaltic wave, peristaltic wave origin, mean peristaltic wave origin, median peristaltic wave origin, one or more features related to the differences between the peristaltic activity in different points of time, entropy, intensity/amplitude root mean square (RMS), first normalized moment, contraction frequency standard deviation, ratio between high and low contraction frequencies, or any combination thereof, and/or any statistical representation of any order of the aforementioned features or any combination thereof. Contraction herein refers to uterine muscle contraction or peristaltic activity of the uterus or the myometrium. Any of the aforementioned features, and any combination thereof may be used to detect a contraction or a contraction event in the received electrical signals from the first and the second electrodes. For example, threshold values for the aforementioned features may be established, such that, a contraction or contraction event is positively detected based on a comparison between the measured or calculated value of the feature and the threshold value established for the feature. Thus, a discrete numerical and/or categorical characterization of the peristaltic activity of the myometrium may be obtained.

The method may further comprise, in parallel or after block 122, and before block 123, receiving, from third and fourth electrodes, electrical signals representative of the electro-physiological state of the myometrium at a point where a second assembly comprising the third and fourth electrodes may be positioned. The method may further comprise, in parallel or after block 122, and before block 123, receiving, from fifth and sixth electrodes, electrical signals representative of the electro-physiological state of the myometrium at a point when a third assembly comprising the fifth and sixth electrodes may be positioned. The method may further comprise, in parallel or after block 122, and before block 123, receiving, from further PPG sensors, signals representative of the blood activity of the myometrium at a point where any of the PPG sensor(s) may be positioned. The method may further comprise, in block 123, identifying a time window with a likelihood, for example the highest likelihood, of assisted reproductive technique success based on the signals received by any one or more of third, fourth, fifth and sixth electrodes and from any of the PPG sensor or further PPG sensors. These methods may be used to detect a contraction or a contraction event in the received electrical signals from any one, two or more of the first, second, third, fourth, fifth and sixth electrodes which may be correlated to the blood activity received from the PPG sensor(s).

In examples, there is disclosed a catheter 10 comprising:
- one or more photoplethysmography, PPG, sensor 15; and
- a first assembly 11 of electrodes, comprising a first electrode 12, the first electrode being a ring sensing electrode, concentrically arranged around the catheter 10; and a second electrode 13, the second electrode being a ring sensing electrode, concentrically arranged around the catheter; wherein the first electrode and the second electrode are separated along a longitudinal axis A of the catheter 10 by a first interelectrode separation; and wherein the second electrode is arranged at a distal separation DS from a distal end 14 of the catheter;
- wherein the PPG sensor 15 is arranged at least between the distal end DS and the first assembly 11;
- the catheter 10 further comprising a lumen 28 configured to receive an internal catheter for passage of an embryo-containing cannula or for direct passage of an embryo or embryos and configured to receive and allow the passage of a liquid substance, wherein an inner diameter of the lumen 28 is equal or greater than 7 FR in the French scale, or equal or greater than 2,33 mm.

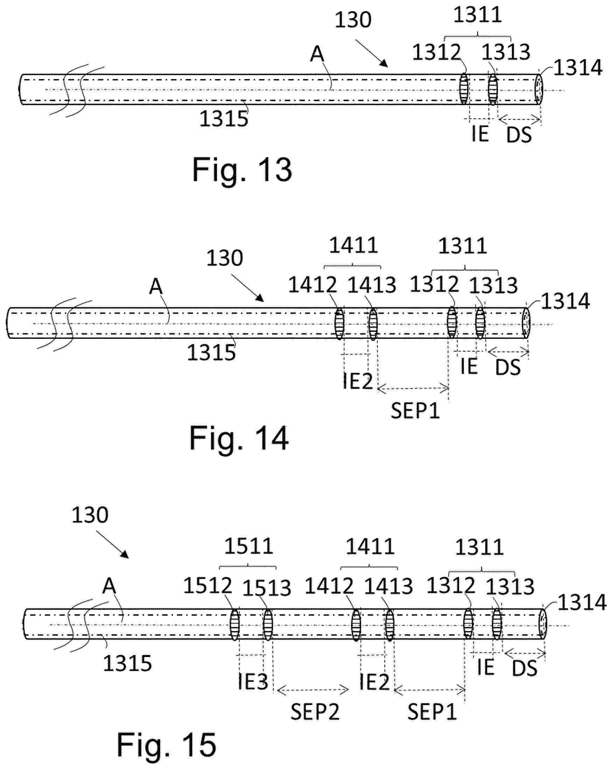

Figure 13 schematically illustrates an assisted reproductive technique catheter, or simply, catheter 130 comprising a lumen 1315 configured to receive an internal catheter for the passage of an embryo-containing cannula or direct passage of an embryo or embryos and configured to receive and allow the passage a liquid substance, wherein the catheter 130 comprises a first assembly 1311 of electrodes, comprising: a first electrode 1312, the first electrode being a ring sensing electrode concentrically arranged around the catheter 130; and a second electrode 1313, the second electrode being a ring sensing electrode concentrically arranged around the catheter 130; wherein the first electrode 1312 and the second electrode 1313 are separated along a longitudinal axis A of the catheter by a first interelectrode separation IE; and wherein the second electrode 1313 is arranged at a distal separation DS from a distal end 1314 of the catheter 1310. The electrodes have a ring portion, which portions may be monolithically formed or separately integrated. The electrical activity sensed by the electrodes may be output via the catheter's wires as an analog signal. Thus, the catheter generates a set of analog signals during a measurement session. A catheter comprising two electrodes, as shown in Figure 13, may generate a set of two analog signals during a measurement session. The analog signals may be transmitted to a display means, such as a monitor or screen, for visualization, which may be referred to as an electromyogram. Analog signals may be transmitted to a processor. In order to transmit the analog signals generated by the electrodes to a processor and/or to visualization means, a connector, for example, a REDEL connector or another suitable connector, may be attached to the catheter. IE may be comprised in a range from 1 mm to 5 mm. DS may be comprised in a range of 0 mm to 10 mm.

Figure 14 schematically illustrates the catheter 130 of figure 13 and further a second assembly 1411 of electrodes, wherein the second assembly 1411 of electrodes comprises at least: a third electrode 1412, the third electrode being a ring sensing electrode concentrically arranged around the catheter 130; and a fourth electrode 1413, the fourth electrode being a ring sensing electrode concentrically arranged around the catheter 130; wherein the third electrode 1412 and the fourth electrode 1413 are separated by a second interelectrode separation IE2; and wherein the second assembly 1411 is arranged at a first separation SEP1 from the first assembly 1311, wherein the first separation SEP1 is comprised in a range from 3 mm to 7 mm. IE may be different from IE2, and IE2 may be comprised in a range from 1 mm to 5 mm. The first separation SEP1 comprises a distance from the fourth electrode 1413, comprised in the second assembly 1411, to the first electrode 1312 comprised in the first assembly 1311. The first separation SEP1 may be configured to detect a contractile wave of interest. The separation between assemblies of electrodes allows sensing points in the uterine cavity which are separated by a controlled or configurable distance, SEP1, so that the obtained signal is representative of the muscular activity of exact points in the uterine cavity.

Figure 15 schematically illustrates the catheter 130 of figure 14 and further comprising a third assembly 1511 of electrodes, wherein the third assembly 1511 of electrodes comprises at least a fifth electrode 1512, the fifth electrode being a ring sensing electrode concentrically arranged around the catheter 130; and a sixth electrode 1513, the sixth electrode being a ring sensing electrode concentrically arranged around the catheter 130; wherein the fifth electrode 1512 and the sixth electrode 1513 are separated by a third interelectrode separation IE3; and wherein the third assembly 1511 is arranged at a second separation SEP2 from the second assembly 1411 of the catheter 130, wherein the second separation SEP2 is comprised in a range from 3 mm to 7 mm. The second separation SEP2 comprises a distance from the sixth electrode 1513, comprised in the third assembly 1511, to the third electrode 1412 comprised in the second assembly 1411.

SEP1 may be different from SEP2. IE3 may be comprised in a range from 1 mm to 5 mm. IE3 may be different from IE and from IE2.

In examples, the first interelectrode separation (IE), and/or the second interelectrode separation (IE2) and/or the third interelectrode separation (IE3), is comprised in a range from 1 mm to 5 mm. The distances may be different from one another.

In examples, at least one of the electrodes is connected by a respective electrical wire to a connector at a proximal end of the catheter 130. The electrical wires may be made of copper, of optical fiber, for example.

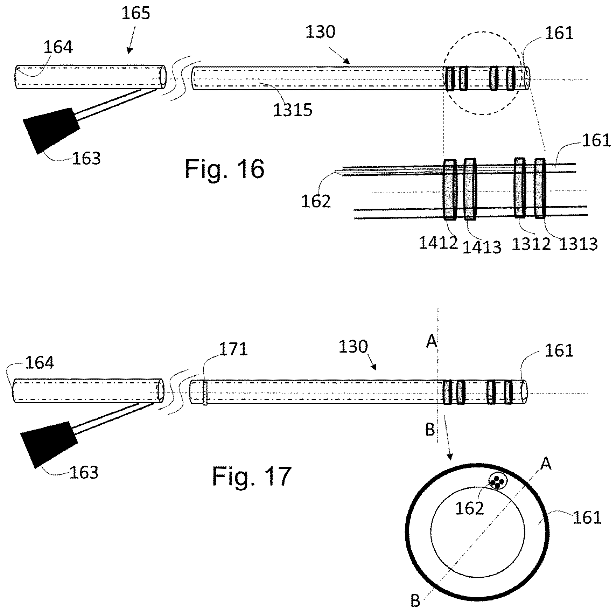

Figure 16 illustrates the catheter 130 of figure 14 further comprising a coating 161, wherein the electrodes 1412, 1413, 1312, 1313 are concentrically arranged around the catheter 130 and over the coating 161; and wherein respective electrical wires in the set of wires 162 is arranged along the catheter 130 from a respective electrode to the connector 163 and is embedded in the coating 161 or arranged between the coating and the catheter. The connector 163 is provided so that the wires can leave the catheter and may connect to an external processing unit. The catheter comprises an entry 164 at a proximal end 165 to insert a cannula or syringe to insert a solution containing reproductive agents into the uterus through the catheter. The entry 164 may comprise flange not shown for attaching an injection syringe or cylinder for the introduction of a solution into the lumen 1315. Figure 16 shows a zoomed portion of the catheter 130 showing the set of wires 162 running embedded inside the coating 161. The figure 16 shows the connector 163 at the proximal end 165 of the catheter 130.

Figure 17 illustrates the example of figure 16 where a front view of the cut along line AB is shown. The set of wires 162 are shown inside a tubular arrangement in the coating 161.

In examples, one or more of the wires may be comprised in between the coating and the catheter or embedded in the coating in a loose manner, or loosely embedded or embedded with certain tolerance to be displaced so that, when the electrodes are displaced for varying any of DS, SEP, SEP2, IE, IE2, the wires may provide a certain tolerance for movement. The length of a wire between an electrode and the connector 1548 may vary and may allow the electrode to be moved in a range of 1mm to 3mm both included.

In examples where DS, SEP, SEP2, IE, IE2, are fixed or predetermined, and the catheter is not configured to move, the wires may be tightly embedded or arranged between the coating and the catheter or the wires may not be allowed to be moved. In those examples, the wires may be tightly embedded or arranged between the coating and the catheter or the wires may also be loosely embedded or embedded with certain tolerance to be displaced.

In some examples the coating is made of a biocompatible material or plastic. The coating may be flexible and the catheter may be flexible.

In examples, as shown in figure 17, the catheter comprises a stopper 171 which is configured to be positioned at the entry of the cervix when in use, configured to stop the catheter and prevent a user to insert the catheter forward during a vaginal insertion of the catheter. The stopper may be positioned at 4 cm or 5 cm from the distal end of the catheter. The stopper may be fixed or may be configured to be displaced from substantially 0,5 cm to 1 cm or 2cm or from 0,5 cm to 5 cm. The stopper 171 may be configured be displaced by application of a displacement force and pressure, for example applied by a user's fingers. The stopper may be displaceable by comprising, for example, an inner helical machining, e.g., in the form of a nut, and the outer surface of the catheter may present, e.g., the form of a screw along the length occupied by the stopper, to displace the stopper screwing the stopper in a direction or an opposite direction depending on the displacement to be obtained. The stopper 171 may be comprised in any of the examples shown in the figures of the disclosure.

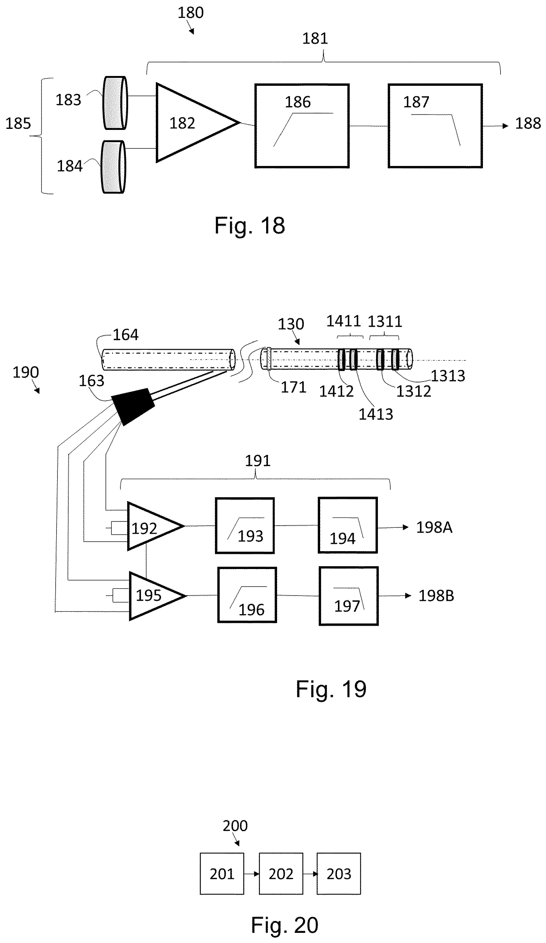

Figure 18 schematically illustrates a system 180 comprising an assembly of electrodes and a bio amplifier 181. For illustrative purposes, the electrodes are shown not arranged around the catheter of the disclosure. In some examples, as illustrated in figure 18, the bio amplifier 181 comprises at least one operational amplifier 182. The bio amplifier processes the two received signals from the electrodes 183 and 184. Figure 18 represents a schematic example of a first electrode 183 of the catheter in electrical connection with a first input pin of the operational amplifier 182 and a second electrode 184 in electrical connection with a second input pin of the operational amplifier 182. The first electrode and the second electrode belong to a same assembly of electrodes 185. In examples, as the one illustrated in figure 18, the system further comprises at least one or more of a high-pass filter 186 and/or a low-pass filter 187 for providing an output signal 188.

Bipolar electrodes provide the advantage of providing located information about the myometrial walls. Monopolar electrodes may provide more generic information of a muscle. The fact of providing assemblies of two bipolar electrodes is that signal processing the signals received from both electrodes may provide information which is more precise in terms of information per mm2 area of a myometrial wall. A bipolar signal is provided by calculating the difference between the voltage amplitude provided by one of the electrodes minus the voltage amplitude provided by the other electrode. The high-pass filter may comprise a cut-off frequency of 0.1 Hz. The low-pass filter may comprise a cut-off frequency of 25Hz.

Figure 19 schematically illustrates a system 190 comprising the catheter 130 comprising two assemblies 1411 and 1311 of electrodes and a bio amplifier 191. The electrodes are shown arranged around a catheter 130. The wires coming from the assembly 1311 may, for example, feed the operational amplifier 192. The result of the difference provided by operational amplifier 192 between the voltage amplitude signals of the two electrodes of the assembly 1311 may be provided to the filter 193, which output may feed a further filter 194. The wires coming from the assembly 1411 may, for example, feed the operational amplifier 195. The result of the difference between the voltage amplitude signals of the two electrodes of the assembly 1411 may be provided to filter 196, which output may feed a further filter 197. The outputs 198A and 198B may be processed to interpret a contractile wave of the myometrial walls for example. Such a contractile wave is represented by the electrical signals provided by the first and second assemblies of electrodes at different heights or points of the uterine cavity. The contractile wave can therefore be represented in two different points of the uterine cavity at the same time. A similar arrangement may be provided for 3 assemblies, and a contractile wave can therefore be represented in three different points of the uterine cavity at the same time. As a contractile wave, theoretically, propagates in a uterine cavity from the fallopian tubes to the cervix, the contractile wave propagation may be followed by the arrangement of assemblies of electrodes in the catheter of the disclosure.

Figure 20 represents an example of a method 200 according to the disclosure for identifying a time of a likelihood, for example a highest likelihood, for assisted reproductive technique success in a subject, the method comprising, in block 201, positioning a catheter within a uterine cavity of the subject, wherein positioning comprises setting the first electrode and the second electrode in contact with at least part of a myometrial wall and/or a myometrial cavity of the subject. In some examples the distance of the first assembly of electrodes from the cervix may be configurable depending on the user or assisted reproductive technique needs. The method comprises, in block 202, receiving, from the first and second electrodes, electrical signals representative of the electro-physiological state of the myometrium; and the method comprises, in block 203, identifying a time when a likelihood, for example a highest likelihood, of assisted reproductive technique success is obtained, based at least in part in the electrical signals.

In some examples, a method for assisted reproductive technique may comprise performing the method identifying a time of highest likelihood for assisted reproductive technique success in a subject and may further comprise, after identifying the time window with the highest likelihood, and before removing the catheter from the uterine cavity, performing the assisted reproductive technique using the catheter during the time with the highest likelihood. The method further comprises, after identifying the time with the highest likelihood, and before removing the catheter from the uterine cavity, performing the assisted reproductive technique using the catheter during a time window comprising at least the time with the highest likelihood, the time window referred to as the time window with the highest likelihood. The time window with the highest likelihood may comprise several minutes, or may comprise several seconds, for example, between 1 minute to 5 minutes. The time window may comprise checking during approximately 2 minutes or 3 minutes that predetermined features are optimum and then, transfer a solution containing reproductive material or agents, for example sperm or an embryo. Some example features may comprise mean frequency, frequency median, standard deviation, ratio of high and low frequencies, first unnormalized moment, RMS, and entropy, or one or more of a contraction frequency, peak contraction intensity, mean contraction intensity, median contraction intensity, median contraction duration, mean contraction duration, peak contraction duration, shortest contraction duration, lowest contraction intensity, direction of propagation of a peristaltic wave, mean direction of propagation of a peristaltic wave, median direction of propagation of a peristaltic wave, peristaltic wave origin, mean peristaltic wave origin, median peristaltic wave origin, one or more features related to the differences between the peristaltic activity in different points of time, entropy, intensity/amplitude root mean square (RMS), first normalized moment, contraction frequency standard deviation, ratio between high and low contraction frequencies, or any combination thereof, and/or any statistical representation of any order of the aforementioned features or any combination thereof. Contraction herein refers to uterine muscle contraction or peristaltic activity of the uterus or the myometrium. Any of the aforementioned features, and any combination thereof may be used to detect a contraction or a contraction event in the received electrical signals from the first and the second electrodes. For example, threshold values for each of the aforementioned features may be established, such that, a contraction or contraction event is positively detected based on a comparison between the measured or calculated value of the feature and the threshold value established for the feature. Thus, a discrete numerical and/or categorical characterization of the peristaltic activity of the myometrium may be obtained.

The method may further comprise, in parallel or after block 202, and before block 203, receiving, from third and fourth electrodes, electrical signals representative of the electro-physiological state of the myometrium at a point where a second assembly comprising the third and fourth electrodes may be positioned. The method may further comprise, in parallel or after block 202, and before block 203, receiving, from fifth and sixth electrodes, electrical signals representative of the electro-physiological state of the myometrium at a point when a third assembly comprising the fifth and sixth electrodes may be positioned. The method may further comprise, in block 203, identifying a time window with the highest likelihood of assisted reproductive technique success based on the signals received by any one or more of third, fourth, fifth and sixth electrodes. These methods may be used to detect a contraction or a contraction event in the received electrical signals from any one, two or more of the first, second, third, fourth, fifth and sixth electrodes.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples but should be determined only by a fair reading of the claims that follow.

## Claims

1. A catheter (10) comprising:
- a first assembly (11) of electrodes, comprising a first electrode (12), the first electrode being a ring sensing electrode, concentrically arranged around the catheter; and a second electrode (12), the second electrode being a ring sensing electrode, concentrically arranged around the catheter; wherein the first electrode and the second electrode are separated along a longitudinal axis of the catheter by a first interelectrode separation (IE); and wherein the second electrode (13) is arranged at a distal separation (DS) from a distal end (14) of the catheter;
- one or more photoplethysmography, PPG, sensors (15, 15a)); wherein the one or more PPG sensors are arranged at least on a surface of the catheter and/or at least embedded in the catheter; and
- **characterized in that** the catheter comprises a lumen (28) configured to receive an internal catheter for passage of an embryo-containing cannula or for direct passage of an embryo or embryos and configured to receive and allow the passage of a liquid substance; wherein an inner diameter of the lumen is equal or greater than 7 FR in the French scale, or equal or greater than 2,33 mm.

2. The catheter (10) of claim 1 further comprising a second assembly (16) of electrodes, wherein the second assembly of electrodes comprises at least:
- a third electrode (17), the third electrode being a ring sensing electrode, concentrically arranged around the catheter; and
- and a fourth electrode (18), the fourth electrode being a ring sensing electrode concentrically arranged around the catheter;
- wherein the third electrode and the fourth electrode are separated by a second interelectrode separation (IE2);
- and wherein the second assembly (16) is arranged at a first separation (SEP1) from the first assembly (11), wherein the first separation is comprised in a range from 3 mm to 7 mm.

3. The catheter (10) of claim 2 further comprising a third assembly (19) of electrodes, wherein the third assembly of electrodes comprises at least:
- a fifth electrode (20), the fifth electrode being a ring sensing electrode concentrically arranged around the catheter; and
- a sixth electrode (21), the sixth electrode being a ring sensing electrode concentrically arranged around the catheter;
- wherein the fifth electrode and the sixth electrode are separated by a third interelectrode separation (IE3);
- and wherein the third assembly is arranged at a second separation (SEP2) from the second assembly of the catheter, wherein the second separation is comprised in a range from 3 mm to 7 mm.

4. The catheter (10) of any one of claims 1 to 3, wherein the first interelectrode separation (IE), and/or the second interelectrode separation (IE2) and/or the third interelectrode separation (IE3), is comprised in a range from 1 mm to 5 mm.

5. The catheter (10) of any one of claims 1 to 4, wherein at least one of the electrodes is connected by a respective electrical wire to a connector (26) at a proximal end of the catheter.

6. The catheter (10) of claim 5 further comprising a coating (22);
- wherein one or more of the electrodes (12, 13, 17, 18) is further arranged around the catheter and over the coating; and
- wherein at least one respective electrical wire is arranged along the catheter from a respective electrode to the connector (26) and is embedded in the coating (22) or arranged between the coating and the catheter.

7. The catheter of claim 6 wherein the coating is made of a biocompatible material or plastic.

8. The catheter of any one of claims 1 to 7, wherein an outer diameter of the electrodes is equal or greater than 7 FR in the French scale or equal or greater than 2,33 mm.

9. The catheter of any one of claims 1 to 8, wherein the distal separation (DS) is a distance between 0 and 10 mm or wherein the distal separation (DS) is at least of 3 mm.

10. The catheter of any one of claims 1 to 9 further comprising a stopper (27).

## Patentansprüche

1. Ein Katheter (10) umfassend:
- eine erste Anordnung (11) von Elektroden, umfassend eine erste Elektrode (12), wobei die erste Elektrode eine Ringerfassungselektrode ist, die konzentrisch um den Katheter herum angeordnet ist; und eine zweite Elektrode (12), wobei die zweite Elektrode eine Ringerfassungselektrode ist, die konzentrisch um den Katheter herum angeordnet ist; wobei die erste Elektrode und die zweite Elektrode entlang einer Längsachse des Katheters durch einen ersten Interelektrodenabstand (IE) getrennt sind; und wobei die zweite Elektrode (13) in einem distalen Abstand (DS) von einem distalen Ende (14) des Katheters angeordnet ist;
- einen oder mehrere PPG-Sensoren (Photoplethysmographiesensoren) (15, 15a); wobei der eine oder die mehreren PPG-Sensoren mindestens auf einer Oberfläche des Katheters angeordnet und/oder mindestens in den Katheter eingebettet sind; und
- **dadurch gekennzeichnet, dass** der Katheter ein Lumen (28) umfasst, das dazu konfiguriert ist, einen inneren Katheter für den Durchgang einer embryohaltigen Kanüle oder für den direkten Durchgang eines Embryos oder von Embryonen aufzunehmen und dazu konfiguriert ist, eine flüssige Substanz aufzunehmen und sein Durchgang zu ermöglichen; wobei ein Innendurchmesser des Lumens gleich oder größer als 7 FR in der französischen Skala oder gleich oder größer als 2,33 mm ist.

2. Der Katheter (10) von Anspruch 1, ferner umfassend eine zweite Baugruppe (16) von Elektroden, wobei die zweite Baugruppe von Elektroden mindestens Folgendes umfasst:
- eine dritte Elektrode (17), wobei die dritte Elektrode eine Ringerfassungselektrode ist, die konzentrisch um den Katheter herum angeordnet ist; und
- und eine vierte Elektrode (18), wobei die vierte Elektrode eine Ringerfassungselektrode ist, die konzentrisch um den Katheter herum angeordnet ist;
- wobei die dritte Elektrode und die vierte Elektrode durch eine zweite Interelektrodenabstand (IE2) getrennt sind;
- und wobei die zweite Baugruppe (16) in einem ersten Abstand (SEP1) von der ersten Baugruppe (11) angeordnet ist, wobei der erste Abstand in einem Bereich von 3 mm bis 7 mm liegt.

3. Der Katheter (10) von Anspruch 2, ferner umfassend eine dritte Baugruppe (19) von Elektroden, wobei die dritte Baugruppe von Elektroden mindestens Folgendes umfasst:
- eine fünfte Elektrode (20), wobei die fünfte Elektrode eine Ringerfassungselektrode ist, die konzentrisch um den Katheter angeordnet ist; und
- eine sechste Elektrode (21), wobei die sechste Elektrode eine Ringerfassungselektrode ist, die konzentrisch um den Katheter herum angeordnet ist;
- wobei die fünfte Elektrode und die sechste Elektrode durch einen dritte Interelektrodenabstand (IE3) getrennt sind;
- und wobei die dritte Baugruppe in einem zweiten Abstand (SEP2) von der zweiten Baugruppe des Katheters angeordnet ist, wobei der zweite Abstand in einem Bereich von 3 mm bis 7 mm liegt.

4. Der Katheter (10) von einem der Ansprüche 1 bis 3, wobei der erste Interelektrodenabstand (IE) und/oder der zweite Interelektrodenabstand (IE2) und/oder der dritte Interelektrodenabstand (IE3) in einem Bereich von 1 mm bis 5 mm liegt.

5. Der Katheter (10) von einem der Ansprüche 1 bis 4, wobei mindestens eine der Elektroden durch einen jeweiligen elektrischen Draht mit einem Verbinder (26) an einem proximalen Ende des Katheters verbunden ist.

6. Der Katheter (10) von Anspruch 5, der ferner eine Beschichtung (22) umfasst;
- wobei eine oder mehrere der Elektroden (12, 13, 17, 18) ferner um den Katheter herum und über der Beschichtung angeordnet ist; und
- wobei mindestens ein jeweiliger elektrischer Draht entlang des Katheters von einer jeweiligen Elektrode zu dem Verbinder (26) angeordnet und in die Beschichtung (22) eingebettet oder zwischen der Beschichtung und dem Katheter angeordnet ist.

7. Der Katheter von Anspruch 6, wobei die Beschichtung aus einem biokompatiblen Material oder Kunststoff hergestellt ist.

8. Der Katheter von einem der Ansprüche 1 bis 7, wobei ein Außendurchmesser der Elektroden gleich oder größer als 7 FR in der französischen Skala oder gleich oder größer als 2,33 mm ist.

9. Der Katheter von einem der Ansprüche 1 bis 8, wobei der distale Abstand (DS) ein Abstand zwischen 0 und 10 mm ist oder wobei der distale Abstand (DS) mindestens 3 mm beträgt.

10. Der Katheter von einem der Ansprüche 1 bis 9, ferner umfassend einen Anschlag (27).

## Revendications

1. Un cathéter (10) comprenant :
- un premier ensemble (11) d'électrodes, comprenant une première électrode (12), la première électrode étant une électrode de détection annulaire, disposée de manière concentrique autour du cathéter ; et une deuxième électrode (12), la deuxième électrode étant une électrode de détection annulaire, disposée de manière concentrique autour du cathéter ; dans lequel la première électrode et la deuxième électrode sont séparées le long d'un axe longitudinal du cathéter par une première séparation interélectrode (IE) ; et dans lequel la deuxième électrode (13) est disposée à une distance distale (DS) d'une extrémité distale (14) du cathéter ;
- un ou plusieurs capteurs de photopléthysmographie, PPG, (15, 15a) ; dans lequel l'un ou les plusieurs capteurs de PPG sont agencés au moins sur une surface du cathéter et/ou au moins intégrés dans le cathéter ; et
- **caractérisé en ce que** le cathéter comprend une lumière (28) configurée pour recevoir un cathéter interne pour le passage d'une canule contenant un embryon ou pour le passage direct d'un embryon ou d'embryons et configurée pour recevoir et permettre le passage d'une substance liquide ; dans lequel un diamètre interne de la lumière est égal ou supérieur à 7 FR dans l'échelle française, ou égal ou supérieur à 2,33 mm.

2. Le cathéter (10) de la revendication 1, comprenant en outre un second ensemble (16) d'électrodes, dans lequel le second ensemble d'électrodes comprend au moins :
- une troisième électrode (17), la troisième électrode étant une électrode de détection annulaire, disposée concentriquement autour du cathéter ; et
- et une quatrième électrode (18), la quatrième électrode étant une électrode de détection annulaire disposée concentriquement autour du cathéter ;
- dans lequel la troisième électrode et la quatrième électrode sont séparées par une deuxième séparation interélectrode (IE2) ;
- et dans lequel le second ensemble (16) est agencé au niveau d'une première séparation (SEP1) du premier ensemble (11), dans lequel la première séparation est comprise dans une plage de 3 mm à 7 mm.

3. Le cathéter (10) de la revendication 2, comprenant en outre un troisième ensemble (19) d'électrodes, dans lequel le troisième ensemble d'électrodes comprend au moins :
- une cinquième électrode (20), la cinquième électrode étant une électrode de détection annulaire disposée concentriquement autour du cathéter ; et
- une sixième électrode (21), la sixième électrode étant une électrode de détection annulaire disposée concentriquement autour du cathéter ;
- dans lequel la cinquième électrode et la sixième électrode sont séparées par une troisième séparation interélectrode (IE3) ;
- et dans lequel le troisième ensemble est agencé au niveau d'une deuxième séparation (SEP2) du deuxième ensemble du cathéter, dans lequel la deuxième séparation est comprise dans une plage de 3 mm à 7 mm.

4. Le cathéter (10) de l'une quelconque des revendications 1 à 3, dans lequel la première séparation interélectrode (IE), et/ou la deuxième séparation interélectrode (IE2) et/ou la troisième séparation interélectrode (IE3), est comprise dans une plage de 1 mm à 5 mm.

5. Le cathéter (10) de l'une quelconque des revendications 1 à 4, dans lequel au moins l'une des électrodes est connectée par un fil électrique respectif à un connecteur (26) au niveau d'une extrémité proximale du cathéter.

6. Le cathéter (10) de la revendication 5 comprenant en outre un revêtement (22) ;
- dans lequel une ou plusieurs des électrodes (12, 13, 17, 18) sont en outre agencées autour du cathéter et sur le revêtement ; et
- dans lequel au moins un fil électrique respectif est agencé le long du cathéter à partir d'une électrode respective jusqu'au connecteur (26) et est intégré dans le revêtement (22) ou agencé entre le revêtement et le cathéter.

7. Le cathéter de la revendication 6, dans lequel le revêtement est fait en un matériau biocompatible ou en plastique.

8. Le cathéter de l'une quelconque des revendications 1 à 7, dans lequel un diamètre extérieur des électrodes est égal ou supérieur à 7 FR dans l'échelle française ou égal ou supérieur à 2,33 mm.

9. Le cathéter de l'une quelconque des revendications 1 à 8, dans lequel la séparation distale (DS) est une distance d'entre 0 et 10 mm ou dans lequel la séparation distale (DS) est d'au moins 3 mm.

10. Le cathéter de l'une quelconque des revendications 1 à 9 comprenant en outre une butée (27).
